(19) Europäisches Patentamt
European Patent Office
Office européen des brevets

(11) EP 2 202 304 B1

(12) EUROPEAN PATENT SPECIFICATION

(45) Date of publication and mention of the grant of the patent:
**17.08.2016 Bulletin 2016/33**

(21) Application number: **08833630.0**

(22) Date of filing: **24.09.2008**

(51) Int Cl.:
***C12N 15/09*** (2006.01) ***C12N 1/15*** (2006.01)
***C12N 1/19*** (2006.01) ***C12N 1/21*** (2006.01)
***C12N 5/10*** (2006.01) ***C12N 9/78*** (2006.01)
***C12Q 1/34*** (2006.01)

(86) International application number:
**PCT/JP2008/067145**

(87) International publication number:
**WO 2009/041409 (02.04.2009 Gazette 2009/14)**

(54) **MODIFIED CREATININE AMIDOHYDROLASE**
MODIFIZIERTE KREATININAMIDOHYDROLASE
CRÉATININE-AMIDOHYDROLASE MODIFIÉE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.09.2007 JP 2007250874**
**27.09.2007 JP 2007250875**
**27.09.2007 JP 2007250876**
**27.09.2007 JP 2007250877**
**27.09.2007 JP 2007250878**

(43) Date of publication of application:
**30.06.2010 Bulletin 2010/26**

(73) Proprietor: **TOYOBO CO., LTD.**
**Osaka-shi**
**Osaka 530-8230 (JP)**

(72) Inventors:
- **HIRAO, Rie**
**Tsuruga-shi**
**Fukui 914-0047 (JP)**
- **KITABAYASHI, Masao**
**Tsuruga-shi**
**Fukui 914-0047 (JP)**
- **NISHIYA, Yoshiaki**
**Tsuruga-shi**
**Fukui 914-0047 (JP)**

(74) Representative: **Vossius & Partner**
**Patentanwälte Rechtsanwälte mbB**
**Siebertstrasse 3**
**81675 München (DE)**

(56) References cited:
**EP-A1- 0 402 929 JP-A- 2008 136 477**
**JP-B2- 2 527 035**

- **YOSHIMOTO TADASHI ET AL: "Crystal structures of creatininase reveal the substrate binding site and provide an insight into the catalytic mechanism." JOURNAL OF MOLECULAR BIOLOGY 19 MAR 2004 LNKD-PUBMED:15003455, vol. 337, no. 2, 19 March 2004 (2004-03-19), pages 399-416, XP004493192 ISSN: 0022-2836**
- **BEUTH BARBARA ET AL: "Crystal structure of creatininase from Pseudomonas putida: a novel fold and a case of convergent evolution." JOURNAL OF MOLECULAR BIOLOGY 5 SEP 2003 LNKD- PUBMED:12946365, vol. 332, no. 1, 5 September 2003 (2003-09-05), pages 287-301, XP004450093 ISSN: 0022-2836**
- **BEUTH, B. ET AL.: 'Crystal structure of creatininase from Pseudomonas putida: a novel fold and a case of convergent evolution.' J MOL BIOL. vol. 332, 2003, pages 287 - 301, XP002605326**

EP 2 202 304 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relates to a modified creatinine amidohydrolase having improved chelating agent resistance.

The present invention further relates to a gene encoding the modified creatinine amidohydrolase; a method for producing the modified creatinine amidohydrolase; and various applications of the modified creatinine amidohydrolase in reagents for measuring creatinine.

The present invention further relates to a method for improving the chelating agent resistance of creatinine amidohydrolase.

furthermore, the present invention relates to a method for improving the specific activity of creatinine amidohydrolase; a modified creatinine amidohydrolase having improved specific activity; a gene encoding the modified creatinine amidohydrolase; a method for producing the modified creatinine amidohydrolase; and various applications of the modified creatinine amidohydrolase in reagents for measuring creatinine.

### BACKGROUND ART

Creatinine amidohydrolase (EC 3.5.2.10) has been used as an enzyme for measuring creatinine in body fluids, which is an indicator used for clinical diagnosis of muscular and renal diseases, together with other enzymes such as creatine amidinohydrolase, sarcosine oxidase, and peroxidase. Creatinine amidohydrolase is an enzyme that acts on creatinine in the presence of water to catalyze a reversible reaction for forming creatine from creatinine.

Such creatinine amidohydrolase is known to be produced by microorganisms of the genera *Pseudomonas* (Non-Patent Document 1) and *Alcaligenes* (Non-Patent Document 2). As other producers of creatinine amidohydrolase, only microorganisms of the genera *Flavobacterium, Corynebacterium, Micrococcus* (Patent Document 1), *Penicillium* (Patent Document 2), etc., are known. A gene that encodes creatinine amidohydrolase produced by *Pseudomonas putida* PS-7 has already been isolated, and the amino acid sequence thereof has been published (Patent Document 3). Previously, the crystal structure of the creatininase from *Pseudomonas putida* has been determined revealing its substrate binding site and providing an insight into its catalytic mechanism; Yoshimoto et al., Journal of Molecular Biology 337(2): 399-416 and Beuth et al., Journal of Molecular Biology 332(1): 287-301.
Further, the creatine aminohydrolase *Pseudomonas putida* and the gene encoding said creatinine amidohydrolase has previously been described; EP 0 402 929 A1.

Non-Patent Document 1: Journal of Biochemistry, Vol. 86, 1109-1117 (1979)
Non-Patent Document 2: Chemical and Pharmaceutical Bulletin, Vol. 34, No. 1, 269-274 (1986)
Patent Document 1: Japanese Unexamined Patent Publication No. 51-115989
Patent Document 2: Japanese Unexamined Patent Publication No. 47-43281
Patent Document 3: Japanese Patent No. 2527035

### DISCLOSURE OF THE INVENTION

### PROBLEM TO BE SOLVED BY THE INVENTION

An object of the present invention is to modify a known creatinine amidohydrolase as described above to provide an enzyme that is more suitable for use in measuring creatinine.

### MEANS FOR SOLVING THE PROBLEM

The creatinine amidohydrolase produced by various known microorganisms has a high Km value for creatinine as an enzyme for clinical test reagents; therefore, this enzyme must be used in large amounts in a clinical test reagent composition. For example, the enzyme derived from *Alcaligenes faecalis* TE3581 (Patent Document 4) is reported to have a Km value for creatinine of about 42 mM. The enzyme derived from *Arthrobacter* sp. TE1826 has a higher Km value for creatinine of about 66 mM (Patent Document 5).

Patent Document 4: Japanese Unexamined Patent Publication No. 9-154574
Patent Document 5: Japanese Unexamined Patent Publication No. 10-215874

In view of the above problem, the present inventors created a modified creatinine amidohydrolase with a low Km value (a modified creatinine amidohydrolase wherein glycine at position 179 is substituted with various amino acids) using a creatinine amidohydrolase gene derived from the *Pseudomonas putida* (PS-7) strain by using a protein engineering method (Japanese Patent Application No. 2006-303451).

However, the modified creatinine amidohydrolase thus obtained has low chelating agent resistance, and therefore has poor resistance to the chelating agents contained in clinical diagnostic reagents or samples, and has poor storage stability. Therefore, the present inventors conducted further research to develop a method for improving the chelating agent resistance of the modified creatinine amidohydrolase.

As a result, the present inventors succeeded in creating a modified creatinine amidohydrolase with improved chelating agent resistance from the above modified creatinine amidohydrolase, another amino acid by further substituting the amino acid at position 175 with another amino acid.

As a result of additional research, the present inventors succeeded in creating a modified creatinine amidohydrolase with improved chelating agent resistance using the above modified creatinine amidohydrolase, wherein glycine at position 179 is substituted with another amino acid, by further substituting the amino acid at position 124 in the amino acid sequence shown in SEQ ID: 2 of the Sequence Listing, or an amino acid at a position equivalent to position 124, with tryptophan.

As a result of further research, the present inventors succeeded in creating a modified creatinine amidohydrolase with improved chelating agent resistance using the above modified creatinine amidohydrolase, wherein glycine at position 179 is substituted with another amino acid, by further substituting the amino acid at position 78 in the amino acid sequence shown in SEQ ID: 2 of the Sequence Listing with threonine or arginine.

As a result of further research, the present inventors succeeded in creating a modified creatinine amidohydrolase with improved chelating agent resistance using the above modified creatinine amidohydrolases, wherein glycine at position 179 is substituted with another amino acid, by further substituting the amino acid at position 122 in the amino acid sequence shown in SEQ ID: 2 of the Sequence Listing with serine.

As described later, apart from the above, the present inventors found that substituting an amino acid at position 43 in a modified creatinine amidohydrolase having a low Km value with aspartic acid or glutamic acid can improve specific activity. The present inventors further found that additionally substituting the amino acid at position 175 in this double mutant can similarly improve the chelating agent resistance.

Further, the present inventors found that even when a wild-type creatinine amidohydrolase is used in place of a modified creatinine amidohydrolase having a low Km value, amino acid substitution at position 175 can provide a modified creatinine amidohydrolase with improved chelating agent resistance.

From the above, the present inventors concluded that amino acid substitution at position 175 is an effective method for improving chelating agent resistance, and accomplished the present invention.

From another viewpoint, the disadvantage of the above modified creatinine amidohydrolase, wherein glycine at position 179 is substituted with another amino acid, is that the protein must be added to a clinical diagnostic reagent at a high concentration because it has a low specific activity. Therefore, the present inventors conducted further research to develop a method for improving the specific activity of the modified creatinine amidohydrolase.

As a result, the present inventors succeeded in creating a modified creatinine amidohydrolase with improved specific activity using the above modified creatinine aminohydrolase, wherein glycine at position 179 is substituted with another amino acid, by further substituting the amino acid at position 43 with aspartic acid or glutamic acid.

As described above, the present inventors found that when a modified creatinine amidohydrolase with a low Km value, particularly a modified creatinine amidohydrolase wherein glycine at position 179 is substituted with serine, is subjected to an amino acid substitution at position 175, the chelating agent resistance is improved. The present inventors further found that substituting an amino acid at position 43 in this double mutant with aspartic acid or glutamic acid can improve the specific activity.

The present inventors found that even when a wild-type creatinine amidohydrolase is used in place of a modified creatinine amidohydrolase with a low Km value, substituting the amino acid at position 43 with aspartic acid or glutamic acid can provide a modified creatinine amidohydrolase with improved activity.

From the above, the present inventors concluded that amino acid substitution at position 43 with aspartic acid or glutamic acid is an effective method for improving the specific activity, and accomplished the present invention.
Thus, the present invention relates to the embodiments characterized in the claims. Thus, it relates to the following items.

1. A modified creatinine amidohydrolase having creatinine amidohydrolase activity wherein:

   (1) the amino acid at position 175 in the amino acid sequence shown in SEQ ID NO: 1 of the Sequence Listing, or at a position equivalent thereto in an amino acid sequence of creatinine amidohydrolase derived from organisms other than Pseudomonas putida (PS-7), is substituted with an amino acid selected from the group consisting of threonine, glutamine, asparagine, isoleucine, serine, and leucine;
   (2) the amino acid at position 175 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), is substituted with an amino acid selected from the group consisting of glutamine, lysine, serine, histidine, leucine, cysteine, glutamic acid, phenylalanine, and tryptophan;

(3) the amino acid at position 175 in the amino acid sequence shown in SEQ ID NO: 3 of the Sequence Listing, or at a position equivalent thereto in an amino acid sequence of creatinine amidohydrolase derived from organisms other than Pseudomonas putida (PS-7), is substituted with an amino acid selected from the group consisting of methionine, glutamine, lysine, serine, threonine, arginine, and histidine;

(4) an amino acid at position 124 in the amino acid sequence shown in SEQ ID NO: 2, or at a position equivalent thereto in an amino acid sequence of creatinine amidohydrolase derived from organisms other than Pseudomonas putida (PS-7), is substituted with tryptophan;

(5) an amino acid at position 78 in the amino acid sequence shown in SEQ ID NO: 2, or at a position equivalent thereto in an amino acid sequence of creatinine amidohydrolase derived from organisms other than Pseudomonas putida (PS-7), is substituted with threonine or arginine;

(6) an amino acid at position 122 in the amino acid sequence shown in SEQ ID NO: 2, or at a position equivalent thereto in an amino acid sequence of creatinine amidohydrolase derived from organisms other than Pseudomonas putida (PS-7), is substituted with serine; or

(7) an amino acid at position 43 in an amino acid sequence shown in at least one of SEQ ID NOS: 1, 2, and 7 to 10, or at a position equivalent thereto in an amino acid sequence of creatinine amidohydrolase derived from organisms other than Pseudomonas putida (PS-7), is substituted with aspartic acid or glutamic acid.

2. A gene encoding the modified creatinine amidohydrolase of item 1.

3. A vector comprising the gene of item 2.

4. A transformant transformed with the vector of item 3.

5. A method for producing a modified creatinine amidohydrolase, comprising culturing the transformant of item 4 and collecting creatinine amidohydrolase from the culture.

6. A reagent for measuring creatinine, comprising the modified creatinine amidohydrolase of item 1.

7. A method for measuring creatinine using the modified creatinine amidohydrolase of item 1.

8. A method for improving the specific activity of creatinine amidohydrolase, comprising substituting an amino acid at position 43 in an amino acid sequence shown in at least one of SEQ ID NOS: 1, 2, and 7 to 10 of the Sequence Listing, or at a position equivalent thereto in an amino acid sequence of creatinine amidohydrolase derived from organisms other than Pseudomonas putida (PS-7), with aspartic acid or glutamic acid.

9. A method for improving the chelating agent resistance of creatinine amidohydrolase, comprising at least one of the following steps (1) to (3):

(1) substituting an amino acid at position 124 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), with tryptophan;

(2) substituting an amino acid at position 78 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), with threonine or arginine; and

(3) substituting an amino acid at position 122 in the amino acid sequence shown in SEQ ID: 2 of the Sequence Listing, or at a position equivalent thereto in an amino acid sequence of creatinine amidohydrolase derived from organisms other than Pseudomonas putida (PS-7), with serine.

Moreover, in the context of the present invention, there is disclosed the following [item 1] to [item 8].

[Item 1]

A modified creatinine amidohydrolase wherein an amino acid at position 175 in an amino acid sequence shown in SEQ ID NO: 1, 2, or 3, or at a position equivalent thereto, is substituted with another amino acid, the modified creatine aminohydrolase having creatinine amidohydrolase activity.

[Item 2]

The modified creatinine amidohydrolase according to item 1 wherein the amino acid at position 175 in the amino acid sequence shown in SEQ ID NO: 1 of the Sequence Listing, or at a position equivalent thereto, is substituted with an amino acid selected from the group consisting of threonine, glutamine, asparagine, isoleucine, serine, and leucine.

[Item 3]

The modified creatinine amidohydrolase according to item 1 wherein the amino acid at position 175 in the amino acid sequence shown in SEQ ID NO: 2 of the Sequence Listing, or at a position equivalent thereto, is substituted with an amino acid selected from the group consisting of glutamine, lysine, serine, histidine, leucine, cysteine, glutamic acid, phenylalanine, and tryptophan.

[Item 4]

The modified creatinine amidohydrolase according to item 1 wherein the amino acid at position 175 in the amino acid sequence shown in SEQ ID NO: 3 of the Sequence Listing, or at a position equivalent thereto, is substituted with an amino acid selected from the group consisting of methionine, glutamine, lysine, serine, threonine, arginine, and histidine.

[Item 5]

A modified creatinine amidohydrolase wherein an amino acid at position 124 in the amino acid sequence shown in SEQ ID NO: 2, or at a position equivalent thereto, is substituted with tryptophan, the modified creatinine amidohydrolase having creatinine amidohydrolase activity.

[Item 6]

A modified creatinine amidohydrolase wherein an amino acid at position 78 in the amino acid sequence shown in SEQ ID NO: 2, or at a position equivalent thereto, is substituted with threonine or arginine, the modified creatinine amidohydrolase having creatinine amidohydrolase activity.

[Item 7]

A modified creatinine amidohydrolase wherein an amino acid at position 122 in the amino acid sequence shown in SEQ ID NO: 2, or at a position equivalent thereto, is substituted with serine, the modified creatinine amidohydrolase having creatinine amidohydrolase activity.

[Item 8]

A modified creatinine amidohydrolase wherein an amino acid at position 43 in an amino acid sequence shown in at least one of SEQ ID NOS: 1, 2, and 7 to 10, or at a position equivalent thereto, is substituted with aspartic acid or glutamic acid, the modified creatinine amidohydrolase having creatinine amidohydrolase activity.

[Item 9]

A gene encoding the modified creatinine amidohydrolase of any one of items 1 to 8.

[Item 10]

A vector comprising the gene of item 9.

[Item 11]

A transformant transformed with the vector of item 10.

[Item 12]

A method for producing a modified creatinine amidohydrolase, comprising culturing the transformant of item 11 and collecting creatinine amidohydrolase from the culture.

[Item 13]

A reagent for measuring creatinine, comprising the modified creatinine amidohydrolase of item 12.

[Item 14]

A method for measuring creatinine using the modified creatinine amidohydrolase of item 12.

[Item 15]

A method for improving the chelating agent resistance of creatinine aminohydrolase, comprising one of the following steps (1) to (4):

(1) substituting an amino acid at position 175 in an amino acid sequence shown in SEQ ID NO: 1, 2, or 3 of the Sequence Listing, or at a position equivalent thereto, with another amino acid;
(2) substituting an amino acid at position 124 in the amino acid sequence shown in SEQ ID NO: 2 of the Sequence Listing, or at a position equivalent thereto, with tryptophan;
(3) substituting an amino acid at position 78 in the amino acid sequence shown in SEQ ID NO: 2 of the Sequence Listing, or at a position equivalent thereto, with threonine or arginine; and
(4) substituting an amino acid at position 122 in the amino acid sequence shown in SEQ ID: 2 of the Sequence Listing, or at a position equivalent thereto, with serine.

[Item 16]

A method for improving the specific activity of creatinine amidohydrolases, comprising substituting an amino acid at position 43 in an amino acid sequence shown in at least one of SEQ ID NOS: 1, 2, and 7 to 10 of the Sequence Listing, or at a position equivalent thereto, with aspartic acid or glutamic acid.

[Item 17]

A method for improving the chelating agent resistance of creatinine amidohydrolase, comprising at least one of the following steps (1) to (4):

(1) substituting an amino acid at position 175 in an amino acid sequence shown in SEQ ID NO: 1, 2, or 3 of the Sequence Listing, or at a position equivalent thereto, with another amino acid; and
(2) substituting an amino acid at position 124 in the amino acid sequence shown in SEQ ID NO: 2 of the Sequence Listing, or at a position equivalent thereto, with tryptophan;
(3) substituting an amino acid at position 78 in the amino acid sequence shown in SEQ ID NO: 2 of the Sequence Listing, or at a position equivalent thereto, with threonine or arginine; and
(4) substituting an amino acid at position 122 in the amino acid sequence shown in SEQ ID: 2 of the Sequence Listing, or at a position equivalent thereto, with serine.

[Item 18]

A method for producing a creatinine amidohydrolase with improved specific activity, comprising substituting an amino acid at position 43 in an amino acid sequence shown in at least one of SEQ ID NOS: 1, 2, and 7 to 10 of the Sequence Listing, or at a position equivalent thereto, with aspartic acid or glutamic acid..

EFFECT OF THE INVENTION

According to the present invention, a novel creatinine amidohydrolase that is useful as an enzyme for clinical diagnostic reagents, has high reactivity and stability, and can be produced on a large industrial scale can be provided.
According to the present invention, a novel creatinine amidohydrolase that is useful as an enzyme for clinical diagnostic reagents, has high specific activity, and can be produced on a large industrial scale can be provided.

BEST MODE FOR CARRYING OUT THE INVENTION

The present invention will be described in detail below.

One embodiment of the present invention is a modified creatinine amidohydrolase wherein an amino acid at position 175 in an amino acid sequence shown in SEQ ID NO: 1, 2, or 3 of the Sequence Listing, or at a position equivalent thereto in an amino acid sequence of creatinine amidohydrolase derived from organisms other than Pseudomonas putida (PS-7), is substituted with another amino acid, the modified creatinine amidohydrolase having creatinine amidohydrolase activity.

Another embodiment of the present invention is a modified creatinine amidohydrolase wherein an amino acid at position 124 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), is substituted with tryptophan, and having creatinine amidohydrolase activity.

Yet another embodiment of the present invention is a modified creatinine amidohydrolase wherein an amino acid at position 78 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), is substituted with threonine or arginine, the modified creatinine amidohydrolase having creatinine amidohydrolase activity.

Still another embodiment of the present invention is a modified creatinine amidohydrolase wherein an amino acid at position 122 in the amino acid sequence shown in SEQ ID NO: 2 of the Sequence Listing, or at a position equivalent thereto n an amino acid sequence of creatinine amidohydrolase derived from organisms other than Pseudomonas putida (PS-7), is substituted with serine, the modified creatinine amidohydrolase having creatinine amidohydrolase activity.

A further embodiment of the present invention is a method for improving the specific activity of creatinine amidohydrolase, comprising substituting an amino acid at position 43 in an amino acid sequence shown in one of SEQ ID NOS: 1, 2, and 7 to 10 of the Sequence Listing, or at a position equivalent thereto in an amino acid sequence of creatinine amidohydrolase derived from organisms other than Pseudomonas putida (PS-7), with aspartic acid or glutamic acid.

A further embodiment of the present invention is a modified creatinine amidohydrolase produced by the above method, i.e., a modified creatinine amidohydrolase wherein an amino acid at position 43 in an amino acid sequence shown in at least one of SEQ ID NOS: 1, 2, and 7 to 10, or at a position equivalent thereto in an amino acid sequence of creatinine amidohydrolase derived from organisms other than Pseudomonas putida (PS-7), is substituted with aspartic acid or glutamic acid, the modified creatinine amidohydrolase having creatinine amidohydrolase activity.

In this specification, each amino acid is expressed by a single letter code or a three letter code. The position of amino acid mutation is expressed in the following manner. For example, "G179S" indicates the substitution of G (Gly) at position 179 with S (Ser), or a modified creatinine amidohydrolase wherein G (Gly) at position 179 is substituted with S (Ser).

Each multiple variant is expressed by joining the above-explained expressions using a plus sign (+). For example, "G179S + D175Q" indicates the substitution of G (Gly) at position 179 with S (Ser) and the substitution of D (Asp) at position 175 with Q (Gin), or a modified creatinine amidohydrolase wherein G (Gly) at position 179 is substituted with S (Ser), and D (Asp) at position 175 is substituted with Q (Gln).

Creatinine amidohydrolase is an enzyme classified as EC 3.5.2.10.

Examples of the creatinine amidohydrolase to be modified to produce the modified creatinine amidohydrolase of the present invention include, but are not limited to, wild-type creatinine amidohydrolase derived from microorganisms of the genera *Corynebacterium, Pseudomonas, Arthrobacter, Flavobacterium, Micrococcus,* and the like.

Specific examples thereof include creatinine amidohydrolase derived from *Pseudomonas putida* (PS-7), whose amino acid sequence is shown in SEQ ID NO: 1, and the gene encoding this amino acid sequence is shown in SEQ ID NO: 4. These sequences are described in Japanese Patent No. 2527035. The amino acids in SEQ ID NO: 1 are numbered by taking methionine as 1.

The creatinine amidohydrolase to be modified to produce the modified creatinine amidohydrolase of the present invention includes those derived from various microorganisms as mentioned above wherein a part of other amino acid residues in the sequence may be deleted, substituted or inserted, or other amino acid residues may be added or substituted, as far as the activity to creatinine is substantially maintained.

The modified creatinine amidohydrolase as disclosed herein include the following embodiments, as far as the activity to creatinine is substantially maintained: a modified creatinine amidohydrolase wherein a tag such as a histidine tag is bound to or inserted into the creatinine amidohydrolase; one wherein another peptide or another protein (e.g., streptavidin or cytochrome) is fused to at least one terminal end of the creatinine amidohydrolase; one wherein the enzyme is chemically modified with a sugar chain or another compound; one wherein the creatinine amidohydrolase is intramolecularly and/or intermolecularly crosslinked by a disulfide bond or the like; and one wherein molecules of the creatinine amidohydrolase are coupled via a linker peptide. Examples of the modified creatinine amidohydrolase of the present invention further include one composed of a combination of fragments of some wild-type creatinine amidohydrolases.

Specific examples of the modified creatinine amidohydrolase to be modified according to the present invention include creatinine amidohydrolase derived from *Pseudomonas putida* (PS-7) wherein glycine at position 179 is substituted with serine; and creatinine amidohydrolase derived from *Pseudomonas putida* (PS-7) wherein asparagine at position 43 is substituted with glutamic acid, and glycine at position 179 is substituted with serine. The amino acid sequences of these modified creatinine amidohydrolases (to be modified to produce the modified creatinine amidohydrolase of the present invention) are shown in SEQ ID NOS: 2 and 3. Typical embodiments of the genes encoding the modified creatinine amidohydrolases are shown in SEQ ID NOS: 5 and 6.

According to one preferable embodiment of the present invention, the modified creatinine amidohydrolase comprises an amino acid sequence of creatinine amidohydrolase derived from *Pseudomonas putida* (PS-7), G179S, or G179S+N43D, wherein aspartic acid at position 175 is substituted with another amino acid.

It is particularly preferable to substitute aspartic acid at position 175 in the amino acid sequence of creatinine amidohydrolase derived from *Pseudomonas putida* (PS-7) with an amino acid selected from the group consisting of threonine, glutamine, asparagine, isoleucine, serine, and leucine.

It is particularly preferable to substitute aspartic acid at position 175 in G179S with an amino acid selected from the group consisting of glutamine, lysine, serine, histidine, leucine, cysteine, glutamic acid, phenylalanine, and tryptophan.

It is particularly preferable to substitute aspartic acid at position 175 in G179S + N43D with an amino acid selected from the group consisting of methionine, glutamine, lysine, serine, threonine, arginine, and histidine.

According to another preferable embodiment of the present invention, the modified creatinine amidohydrolase comprises an amino acid sequence of creatinine amidohydrolase derived from *Pseudomonas putida* (PS-7) or G179S, wherein glutamic acid at position 124 is substituted with tryptophan.

According to another preferable embodiment of the present invention, the modified creatinine amidohydrolase comprises an amino acid sequence of creatinine amidohydrolase derived from *Pseudomonas putida* (PS-7) or G179S, wherein serine at position 78 is substituted with arginine.

According to another preferable embodiment of the present invention, the modified creatinine amidohydrolase comprises an amino acid sequence of creatinine amidohydrolase derived from *Pseudomonas putida* (PS-7) or G179S, wherein glutamic acid at position 122 is substituted with serin.

According to another embodiment of the present invention, the modified creatinine amidohydrolase of the present invention includes a modified creatinine amidohydrolase comprising an amino acid sequence of creatinine amidohydrolase derived from *Pseudomonas putida* (PS-7) wherein aspartic acid at position 175 is substituted with glutamine, and glycine at position 179 is substituted with serine; one wherein aspartic acid at position 175 is substituted with lysine, and glycine at position 179 is substituted with serine; one wherein aspartic acid at position 175 is substituted with serine, and glycine at position 179 is substituted with serine; and one wherein aspartic acid at position 175 is substituted with histidine, and glycine at position 179 is substituted with serine. These amino acid sequences are shown in SEQ ID NOS: 7 to 10. Typical embodiments of the genes encoding these enzymes are shown in SEQ ID NOS: 11 to 14.

It is particularly preferable to substitute asparagine at position 43 in at least one amino acid sequence selected from the group consisting of the amino acid sequence of creatinine amidohydrolase derived from *Pseudomonas putida* (PS-7), G179S, G179S + D175Q, G179S + D175K, G179S + D175S, and G179S + D175H, with aspartic acid or glutamic acid.

The above substitution position may be an equivalent position in an amino acid sequence of creatinine amidohydrolase derived from organisms other than Pseudomonas putida (PS-7). Whether or not a position is equivalent can be determined based on knowledge of the primary and tertiary structure of the enzyme.

Although the tertiary structure of creatinine amidohydrolase derived from *Pseudomonas putida* (PS-7) was elucidated, there was no description suggesting that the chelating agent resistance or specific activity of creatinine amidohydrolase could be improved by a protein-engineering technique (Non-Patent Documents 3 and 4).

Non-Patent Document 3: Journal of Molecular Biology, Vol 337, 399-416 (2004)
Non-Patent Document 4: Journal of Molecular Biology, Vol 332, 287-301 (2004)

The modified creatinine amidohydrolase as disclosed herein include those as mentioned above wherein a part of other amino acid residues in the sequence may be further deleted, substituted or inserted, or other amino acid residues.may be added or substituted, as far as the effect of the present invention is substantially maintained. The modified creatinine amidohydrolase as disclosed herein may include the following embodiments, as far as the activity to creatinine is substantially maintained: a modified creatinine amidohydrolase wherein a tag such as a histidine tag is bound to or inserted into the creatinine amidohydrolase; one wherein another peptide or another protein (e.g., streptavidin or cytochrome) is fused to at least one terminal end of the creatinine amidohydrolase; one wherein the enzyme is chemically modified with a sugar chain or another compound; one wherein the creatinine amidohydrolase is intramolecularly and/or intermolecularly crosslinked by a disulfide bond or the like; and one wherein molecules of the creatinine amidohydrolase are coupled via a linker peptide. The modified creatinine amidohydrolase as disclosed herein may be one composed of a combination of fragments of some wild-type creatinine amidohydrolases.

The present invention further includes a gene encoding the modified creatinine amidohydrolase.

The gene encoding the modified creatinine amidohydrolase of the present invention can be obtained, for example, by modifying a DNA fragment containing a gene encoding a wild-type creatinine amidohydrolase obtained from various sources (origins) such as microorganisms. Specific examples thereof include bacteria such as Alcaligenes faecalis, Arthrobacter sp., Flavobacterium sp., Corynebacterium ureafaciens, Corynebacterium creatinovorans, Micrococcus luteus, Pseudomonas putida, and the like.

The gene encoding the modified creatinine amidohydrolase as disclosed herein preferably hybridizes with a DNA having a base sequence complementary to the base sequence represented by any of SEQ ID NOS: 4 to 6, and 11 to 14 under stringent conditions and encodes a protein having creatinine amidohydrolase activity.

The gene as disclosed herein may also include a gene obtained by further modification of codon usage in a gene encoding a modified creatinine amidohydrolase that was obtained by modification of a gene encoding a wild-type creatinine amidohydrolase so that the expression of the creatinine amidohydrolase is improved.

As a method for modifying the gene encoding a wild-type creatinine amidohydrolase, a conventional method for modifying genetic information can be employed. That is, a DNA having the genetic information of a modified protein is prepared by modifying a particular base of the DNA having the genetic information of the protein, or inserting a particular base into or deleting a particular base from the DNA. Specific examples of the method for modifying a base in DNA include, for example, use of a commercially available kit (e.g., the Transformer Site-Directed Mutagenesis Kit produced by Clonetech; and the Quick Change Site Directed Mutagenesis Kit produced by Stratagene) or use of a polymerase chain reaction (PCR) method.

The present invention also includes a vector containing a gene encoding the modified creatinine amidohydrolase, and further, a transformant transformed with the vector.

The DNA having the genetic information of the modified protein thus prepared is connected to a plasmid and introduced into a host microorganism to obtain a transformant producing the modified protein.

For example, when Escherichia coli is used as a host microorganism, a plasmid such as pBluescript or pUC18 can be used as the vector. Examples of the host microorganisms that can be used include Escherichia coli W3110, Escherichia coli C600, Escherichia coli JM109, Escherichia coli DH5$\alpha$ and the like. When the host microorganism is a microorganism that belongs to the Escherichia genus, examples of methods for introducing the recombinant vector into the host microorganism may include a method in which recombinant DNA is introduced in the presence of calcium ions, and an electroporation method. Further, a commercially available competent cell (e.g., Competent High JM109 produced by Toyobo Co., Lid.) may be used.

Such a gene may be extracted from these strains or synthesized chemically. Further, DNA fragments containing the creatinine amidohydrolase gene can be obtained by the use of a PCR method.

In the present invention, a gene encoding the creatinine amidohydrolase may be obtained by the following method: For example, a chromosome derived from the *Pseudomonas* putida (PS-7) strain is isolated and purified, and then the DNA is cleaved by ultrasonication, restriction enzyme treatment, or the like. Using a DNA ligase or the like, the cleaved DNA is ligated to a linear expression vector at the blunt ends or the cohesive ends of the DNA, followed by sealing, to construct a recombinant vector. A microorganism carrying the recombinant vector containing the gene encoding the creatinine amidohydrolase is obtained by introducing the recombinant vector into a replicable host microorganism and screening by using a vector marker and the expression of enzyme activity as indicators.

Then, the microorganism carrying the above-mentioned recombinant vector is cultured, and the recombinant vector is isolated and purified from the cultured microorganism. Thereby, a gene encoding the creatinine amidohydrolase can be collected from the expression vector. Specifically, for example, the chromosomal DNA of Pseudomonas putida (PS-7), i.e., the gene donor, can be collected in the following manner.

The culture liquid obtained by culturing the gene-donor microorganism with stirring, for example, for 1 to 3 days is centrifuged to collect the bacterial cells, and these bacterial cells are subjected to lysis to prepare a lysate containing the creatinine amidohydrolase gene. As a lysis method, for example, a treatment with a lytic enzyme, such as lysozyme is carried out, and enzymes such as protease and surfactants such as sodium dodecylsulfate (SDS) may be used in combination, if necessary. Further, a physical pulverization means such as freeze thawing or the French press treatment may be used in combination.

Isolation and purification of a DNA from the lysate thus obtained can be carried out, for example, by appropriately combining, according to conventional methods, treatments such as the removal of protein using phenol, or protease treatment, with methods such as ribonuclease treatment, an alcohol-precipitation treatment, and the like.

Cleavage of the DNA that is isolated and purified from the microorganism can be carried out, for example, by ultrasonication, a restriction enzyme treatment, or the like. Preferably, a type II restriction enzyme that acts on a specific nucleotide sequence is employed.

As the vector for cloning, a vector constructed for gene recombination from a phage or plasmid capable of autonomous replication in the host microorganism is suitable. Examples of such phages include Lambda gt10, Lambda gt11, and the like, for example, when Escherichia coli is used as the host microorganism. Further, examples of the plasmids include pBR322, pUC19, pBluescript, and the like, for example, when Escherichia coli is used as the host microorganism.

A vector fragment can be obtained during cloning by cleaving a vector with a restriction enzyme used for the cleavage of the microbial DNA, i.e., the donor of the gene encoding the creatinine amidohydrolase mentioned above; however, it is not essential to use the same restriction enzyme as used for the cleavage of the microbial DNA. The method for ligating the microbial DNA fragment with the vector DNA fragment is not limited insofar as a known DNA ligase is used. For example, a recombinant vector consisting of the microbial DNA fragment and the vector DNA fragment can be prepared using a suitable DNA ligase after the cohesive end of the microbial DNA fragment is annealed to the cohesive end of the vector fragment. If necessary, after annealing, the resultant can be introduced into a host microorganism to prepare a recombinant vector using DNA ligase in vivo.

The host microorganism used in cloning is not particularly limited insofar as the recombinant vector therein is stable, can proliferate autonomously, and allows phenotypic expression of exogenous genes. General examples thereof include Escherichia coli W3110, Escherichia coli C600, Escherichia coli HB101, Escherichia coli JM109, Escherichia coli DH5α, and the like.

As a method for introducing the recombinant vector into the host microorganism, a competent cell method using a calcium treatment, an electroporation method, or the like may be employed, when the host microorganism is Escherichia coli.

The transformant microorganism thus obtained can stably produce a large amount of the creatinine amidohydrolase when cultured in a nutrient medium. Selection of the target recombinant vector for introduction into the host microorganism can be carried out by screening for microorganisms that express a drug resistance marker of the vector containing the target DNA.

The base sequence of the creatinine amidohydrolase gene obtained by the above-mentioned method was decoded by the dideoxy method described in Science, Vol. 214, 1205 (1981). In addition, the amino acid sequence of the creatinine amidohydrolase was deduced from the base sequence thus determined.

The creatinine amidohydrolase gene contained in the recombinant vector selected as mentioned above can be easily incorporated into a recombinant vector that can be replicated in a microorganism having a creatinine amidohydrolase-producing capability by recovering the DNA of the creatinine amidohydrolase gene from the recombinant vector containing the creatinine amidohydrolase gene by using a restriction enzyme and a PCR method, and ligating the recovered DNA with another vector fragment. Transformation of the microorganism having creatinine amidohydrolase-producing capability with such a vector can be carried out, for example, by a competent cell method using a calcium treatment or an electroporation method.

The present invention also relates to a method for producing a modified creatinine amidohydrolase, comprising culturing a transformant transformed with a vector containing a gene encoding the modified creatinine amidohydrolase.

For example, the transformant microorganism thus obtained can stably produce a large amount of the modified protein when cultured in a nutrient medium. The culture conditions of the transformant, i.e., the host microorganism, may be determined by taking into account the nutritional physiological properties of the host microorganism. A liquid culture medium is used in most cases. Aeration-agitation culture is advantageous in terms of industrial applications.

A wide variety of nutrients commonly used for microbial culture are used as nutrient sources of the culture medium. Any assimilable carbon compounds can be used as a carbon source. Examples thereof include glucose, sucrose, lactose, maltose, lactose, molasses, pyruvic acid, and the like. Further, any available nitrogen compounds can be used as a nitrogen source. Examples thereof include peptone, meat extract, yeast extract, casein hydrolysate, soybean cake alkali extract, and the like. In addition, a phosphate salt, a carbonate salt, a sulfate salt, and salts of magnesium, calcium, potassium, iron, manganese and zinc, particular amino acids, particular vitamins, and the like are used, according to necessity.

The cultivation temperature can be varied appropriately in a range that allows the growth of bacteria and the production of the modified creatinine amidohydrolase. However, in the case of a microorganism having creatinine amidohydrolase-producing capability, the temperature is preferably about 20°C to about 42°C. The cultivation period varies to some extent according to conditions. Cultivation is appropriately terminated by choosing a time when the modified creatinine amidohydrolase reaches a maximum yield. Usually, the cultivation is carried out for about 6 to 48 hours. The pH of the culture medium can vary appropriately in a range that allows the growth of bacteria and the production of the modified creatinine amidohydrolase. Preferably, the pH is in a range of about 6.0 to about 9.0.

The culture liquid containing the modified creatinine amidohydrolase-producing microorganism in the culture can be collected *as is* and used. In general, when the modified creatinine amidohydrolase is present in the culture liquid, the culture liquid is separated into a solution containing the modified creatinine amidohydrolase and the microorganism by filtration, centrifugation, or the like according to a conventional method; and the resulting solution is used. If the modified creatinine amidohydrolase is present in the bacterial cells, the bacterial cells are collected from the culture that is obtained by employing filtration, centrifugation, or the like, and then ruptured by a mechanical method, an enzymatic method using lysozyme, or the like, and, if necessary, adding a chelating agent such as EDTA and a surfactant thereto to make the creatinine amidohydrolase soluble. Thereby, the creatinine amidohydrolase can be separately obtained as an aqueous solution.

The creatinine amidohydrolase-containing solution thus obtained can be, for example, concentrated under vacuum or with membrane filters; salted out with ammonium sulfate, sodium sulfate, or the like; or precipitated by fractional precipitation using a hydrophilic organic solvent such as methanol, ethanol, acetone, or the like. A heat treatment and an isoelectric point treatment are also effective purification processes. Then, the purified creatinine amidohydrolase can be obtained by carrying out gel filtration using an adsorbent or a gel filtration agent, adsorption chromatography, ion exchange chromatography, or affinity chromatography.

For example, the purified creatinine amidohydrolase can be separated and purified by gel filtration using Sephadex gel (GE Healthcare Biosciences) or column chromatography using DEAE Sepharose CL-6B (GE Healthcare Biosciences), octyl Sepharose CL-6B (GE Healthcare Biosciences), or the like, thereby obtaining a purified enzyme preparation. Preferably, the purified enzyme preparation is purified to such an extent that a single band is observed during electrophoresis (SDS-PAGE).

The purified enzyme thus obtained can be made into a powder, for example, by freeze drying, vacuum drying, or spray drying, and distributed. In this case, the purified enzyme can be used by dissolving it in a phosphate buffer solution, a Tris hydrochloride buffer solution, or a Good's buffer solution. Good's buffer solutions are preferred, with PIPES, MES, or MOPS buffer solution being particularly preferred. The creatinine amidohydrolase can be further stabilized by addition of an amino acid such as glutamic acid, glutamine, or lysine, and other additives such as serum albumin.

The method for producing the modified protein of the present invention is not particularly limited, but the modified protein can be produced by the following procedure. A conventional method for modifying genetic information can be employed as a method for modifying an amino acid sequence constituting a protein. That is, a DNA containing the genetic information of the modified protein is prepared by changing a particular base of the DNA carrying the genetic information of the protein, or by inserting or deleting a particular base thereof. Specific examples of the methods for modifying a base in DNA include the use of a commercially available kit (Transformer Mutagenesis Kit manufactured by Clonetech, EXOIII/Mung Bean Deletion Kit manufactured by Stratagene, Quick Change Site Directed Mutagenesis Kit manufactured by Stratagene), etc., or the use of a polymerase chain reaction (PCR) method.

In the present invention, position 175 of the amino acid sequence of the creatinine amidohydrolase shown in SEQ ID NO: 1, 2, or 3 was focused upon. Mutants were introduced at random at these amino acid sites by using the above-mentioned mutagenesis kit to create a library, and then screened using a change in resistance to chelating agents as an indicator. As a result, a modified creatinine amidohydrolase having improved chelating agent resistance was obtained.

In the present invention, position 124 of the amino acid sequence of the creatinine amidohydrolase shown in SEQ ID NO: 2 was focused upon. Mutants were introduced at random at these amino acid sites by using the above-mentioned mutagenesis kit to create a library, and then screened using a change in resistance to chelating agents as an indicator. As a result, a modified creatinine amidohydrolase having improved chelating agent resistance was obtained.

In the present invention, position 78 of the amino acid sequence of the creatinine amidohydrolase shown in SEQ ID NO: 2 was focused upon. Mutants were introduced at random at these amino acid sites by using the above-mentioned mutagenesis kit to create a library, and then screened using a change in resistance to chelating agents as an indicator. As a result, a modified creatinine amidohydrolase having improved chelating agent resistance was obtained.

In the present invention, position 122 of the amino acid sequence of the creatinine amidohydrolase shown in SEQ ID NO: 2 was focused upon. Mutants were introduced at random at these amino acid sites by using the above-mentioned mutagenesis kit to create a library, and then screened using a change in resistance to chelating agents as an indicator. As a result, a modified creatinine amidohydrolase having improved chelating agent resistance was obtained.

In the present invention, position 43 of the amino acid sequence of the creatinine amidohydrolase shown in SEQ ID NOS: 1, 2, and 7 to 10 was focused upon. Mutants were introduced at random at these amino acid sites by using the above-mentioned mutagenesis kit to create a library, and then screened using a change in specific activity as an indicator. As a result, a modified creatinine amidohydrolase having improved specific activity was obtained.

Another embodiment of the present invention includes a reagent for measuring creatinine containing the modified creatinine amidohydrolase according to any one of the items described above.

Embodiments of the reagent are not particularly limited. The reagent can be in the form of a composition for measuring creatinine and/or a kit for measuring creatinine.

In each embodiment described above, the modified creatinine amidohydrolase, the composition for measuring creatinine, and the kit for measuring creatinine of the present invention can be made in various forms such as liquids (aqueous solution, suspension, etc.), powders, freeze dried products, etc. The freeze drying method is not particularly limited: it can be carried out by a conventional method. The composition containing the enzyme of the present invention is not limited to a freeze dried product: it may be in the form of a solution obtained by re-dissolving a freeze dried product.

Further, in each embodiment described above, the modified creatinine amidohydrolases, the composition for measuring creatinine, and the kit for measuring the creatinine of the present invention may be in a purified state according to its form and application, and if necessary, other various additives such as surfactants, stabilizers, excipients, etc., may be added.

The method for blending such additives into the present invention is not particularly limited. Examples thereof include a method for blending a stabilizer into a creatinine amidohydrolase-containing buffer solution, a method for blending creatinine amidohydrolase into a stabilizer-containing buffer solution, a method of simultaneously blending creatinine amidohydrolase and a stabilizer into a buffer solution, and the like.

Although the buffer solution contained is not particularly limited, preferable are a Tris buffer solution, a phosphate buffer solution, a borate buffer solution, a Good's buffer solution, and the like. The pH of the buffer solution is adjusted in a range of about 5.0 to 10.0 according to the intended use. The content of the buffer in the freeze dried product is not particularly limited, but it is used in the amount of 0.1% (weight ratio) or more, particularly preferably in the range of 0.1 to 30% (weight ratio).

In addition, serum albumin may be added. When serum albumin is added to the above-mentioned aqueous composition, the content thereof is preferably 0.05 to 0.5 wt.%.

Examples of the albumin that can be used include bovine serum albumin (BSA), ovalbumin (OVA), and the like. BSA is particularly preferable. The content of the albumin is preferably in a range of 1 to 80% (weight ratio), more preferably 5 to 70% (weight ratio).

On the other hand, in each embodiment described above, the modified creatinine amidohydrolase, the composition for measuring creatinine, and the kit for measuring creatinine of the present invention can be formed in a structure that does not contain protein components other than host-derived protein components.

Examples of protein components other than host-derived protein components include bio-derived materials such as BSA.

Such a structure may possibly reduce non-specific reactions in the creatinine measurement system.

Any buffer that is conventionally used may be used. Usually, a buffer capable of adjusting the pH of the composition to 5 to 10 is preferred. More preferable examples of buffers include buffers such as boric acid and acetic acid, and Good's buffers such as BES, Bicine, Bis-Tris, CHES, EPPS, HEPES, HEPPSO, MES, MOPS, MOPSO, PIPES, POPSO, TAPS, TAPSO, TES, and Tricine.

The content of the buffer (W/W) in a powder composition is preferably 1.0% to 50%.

In addition, an amino acid or an organic acid may be added to a composition essentially comprising the modified creatinine amidohydrolase and a buffer. The composition may be an aqueous composition or a freeze dried product insofar as it contains these components.

Another embodiment of the present invention includes a method for measuring creatinine with the use of the modified creatinine amidohydrolase according to any one of the items described above.

As described in the examples below, the modified creatinine amidohydrolase of the present invention has improved chelating agent resistance. This, for example, improves the preservation stability of clinical diagnostic agents, and extends the expiration date.

There is also disclosed a method for improving the resistance of creatinine amidohydrolase to chelating agents, comprising substituting an amino acid at position 175 of an amino acid sequence represented by SEQ ID NO. 1, 2, or 3 of the Sequence Listing, or at a position equivalent thereto, with other amino acid(s).

Further, there is also disclosed a method for producing creatinine amidohydrolase having improved chelating agent resistance, comprising substituting an amino acid at position 175 of an amino acid sequence represented by SEQ ID NO. 1, 2, or 3 of the Sequence Listing, or at a position equivalent thereto, with other amino acid(s).

The present invention is also a method for improving the resistance of creatinine amidohydrolase to chelating agents, comprising substituting an amino acid at position 124 of an amino acid sequence represented by SEQ ID NO. 2 of the Sequence Listing, or at a position equivalent thereto in an amino acid sequence of creatinine amidohydrolase derived from organisms other than Pseudomonas putida (PS-7), with tryptophan.

There is also disclosed a method for producing creatinine amidohydrolase having improved chelating agent resistance, comprising substituting an amino acid at position 124 of an amino acid sequence represented by SEQ ID NO. 2 of the Sequence Listing, or at a position equivalent thereto, with tryptophan.

The present invention is also a method for improving the resistance of creatinine amidohydrolase to chelating agents, comprising substituting serine at position 78 of an amino acid sequence represented by SEQ ID NO. 2 of the Sequence Listing, with threonine or arginine.

There is also disclosed a method for producing creatinine amidohydrolase having improved chelating agent resistance, comprising substituting serine at position 78 of an amino acid sequence represented by SEQ ID NO. 2 of the Sequence Listing, with threonine or arginine.

The present invention is also a method for improving the resistance of creatinine amidohydrolase to chelating agents, comprising substituting an amino acid at position 122 of an amino acid sequence represented by SEQ ID NO. 2 of the Sequence Listing, or at a position equivalent thereto in an amino acid sequence of creatinine amidohydrolase derived from organisms other than Pseudomonas putida (PS-7), with serine.

Furthermore, there is also disclosed a method for producing creatinine amidohydrolase having improved chelating agent resistance, comprising substituting an amino acid at position 122 of an amino acid sequence represented by SEQ ID NO. 2 of the Sequence Listing, or at a position equivalent thereto, with serine.

There is also disclosed a method for producing creatinine amidohydrolase having improved specific activity, comprising substituting an amino acid at position 43 of amino acid sequences represented by any one of SEQ ID NOS. 1, 2, and 7 to 10 of the Sequence Listing, or at a position equivalent thereto, with asparaginic acid or glutamic acid.

Further, there is also disclosed method for producing a composition for measuring creatinine having improved measurement response, comprising including the amino acid-modified creatinine amidohydrolase according to any one of the items described above in a creatinine measurement system that uses creatinine amidohydrolase.

Specifically, the method of the present invention for improving the resistance of the creatinine amidohydrolase to chelating is to strongly bind metal that is coordinated to the creatinine amidohydrolase. It denotes a method for improving stability.

Examples of chelating agents of the present invention include EDTA, NTA, PDTA, and the like. Although EDTA was used for evaluation as a typical chelating agent, the effect can also be obtained when a chelating agent other than EDTA is used.

Resistance to chelating agents was measured as follows.

The enzyme solution is divided into two parts. EDTA· 3Na is added to one part but not the other. These parts are heat treated for a certain period of time, and the creatinine amidohydrolase activity in both parts is measured using the method described below. The chelating agent resistance is determined as follows: (activity with addition of chelating agent)/(activity without addition of chelating agent)x100(%).

EXAMPLES

Hereinafter, the present invention is described in more detail with reference to Examples, but is not limited to the Examples.

Example 1: Preparation of modified creatinine amidohydrolase gene

In accordance with methods disclosed in Japanese Patent No. 2527035 and Biosci. Biotech. Biochem., Vol. 59, No. 7, pp. 1331-1332 (1995), the chromosomal DNA of the *Pseudomonas putida* PS-7 strain was prepared, and an expression plasmid pCNH5-13 containing a creatinine amidohydrolase gene that originated from the above strain was prepared.

The expression plasmid pCNH5-13 of a wild-type creatinine amidohydrolase is a plasmid obtained by inserting a structural gene encoding creatinine amidohydrolase originated from the *Pseudomonas putida* (PS-7) strain into the multicloning site of Vector pBluescript SK(-). The nucleotide sequence of the above is represented by SEQ ID NO: 4 of the Sequence Listing; and the amino acid sequence of creatinine amidohydrolase deduced from the above nucleotide sequence is represented by SEQ ID NO: 1 of the Sequence Listing.

Subsequently, mutation was performed by using a synthetic oligonucleotide (about 40 mer) containing pCNH5-13 and a triplet coding for an amino acid at the mutation introduction site in the middle of its sequence, using the QuickChange™ Site-Directed Mutagenesis Kit (product of Stratagene) in accordance with its protocol; thereby, a mutation library in which an amino acid substitution mutation was randomly introduced at the 175th position was constructed. Then, a candidate strain was obtained by screening using a change in EDTA resistance as an index, the nucleotide sequence of the candidate strain was determined, and a recombinant plasmid (pCNH-D175T) encoding a mutant creatinine amidohydrolase in which the aspartic acid at the 175th position in the amino acid sequence represented by SEQ ID NO: 1 was substituted with threonine was thus obtained.

Library production and screening were repeated in the same manner as above to obtain recombinant plasmids, each encoding a mutant creatinine amidohydrolase having an improved chelating agent resistance.

Example 2: Preparation of modified creatinine amidohydrolase

Escherichia coli DH5α competent cells were transformed with each of the recombinant plasmids and each of the corresponding transformants were obtained.

Five milliliters of CNH production medium (1% polypeptone, 2% yeast extract, 1% sodium chloride, and 5 mM manganese chloride) was introduced into a test tube, and then autoclaved at 121°C for 20 minutes. After allowed to cool down, ampicillin separately sterilized by filtration was added thereto to a concentration of 100 μL/mL. Into the resulting medium, a single colony of Escherichia coli DH5α (pCNH-D175T) preliminarily cultured at 37°C for 16 hours in an LB agar medium containing 100 μL/mL of ampicillin was inoculated, and then an aeration culture was conducted under stirring at 37°C for 22 hours.

The above bacterial cells were harvested by centrifugal separation, suspended in a 20 mM potassium phosphate buffer solution (pH 7.8), disrupted by ultrasonication, and further subjected to centrifugal separation to thereby obtain a supernatant liquid as a crude enzyme solution. The mutant thereof was designated as D175T. The same procedure was repeated as above to obtain each crude enzyme solution.

Comparative Example 1: Preparation of wild-type creatinine amidohydrolase

In Comparative Example 1, Escherichia coli DH5α transformant obtained by using pCNH5-13 were subjected to the same procedure as above to obtain crude enzyme solution.

Comparative Example 2: Preparation of creatinine amidohydrolase (G179S)

In Comparative Example 2, Escherichia coli DH5α transformants obtained by using pCNH-G179S were subjected to the same procedure as above to obtain crude enzyme solution.

Comparative Example 3: Preparation of creatinine amidohydrolase (G179S+N43D)

In Comparative Example 3, Escherichia coli DH5α transformants obtained by using pCNH-G179S+N43D were subjected to the same procedure as above to obtain crude enzyme solution.

Example 3: Evaluation of modified creatinine amidohydrolase 1

The mutant creatinine amidohydrolase obtained in Example 2 and the various creatinine amidohydrolase (D175X) obtained in Comparative Example 1 were independently added to 20 mM potassium phosphate buffer solutions (pH 7.8), each of which was then divided into 2 portions. EDTA-3Na was added to one of the portions to a final concentration of 5 mM, while nothing was added to the other, and the 2 portions were treated at 60°C for 20 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Table 1 shows the results. As is clear from Table 1, the modified creatinine amidohydrolase of the present invention showed an improved chelating agent resistance, compared with that before modification.

Table 1

| Amino Acid X | EDTA Resistance (%) | 11 mM/55mM ratio | Specific Activity (U/A280) |
|---|---|---|---|
| | 50.1 | 0.32 | 350 |
| T | 80.7 | 0.39 | - |
| Q | 74.1 | 0.43 | - |
| N | 66.1 | 0.31 | - |
| I | 65.3 | 0.43 | - |
| S | 64.2 | 0.37 | - |
| L | 64.2 | 0.38 | - |

Example 4: Evacuation of modified creatinine amidohydrolase 2

The mutant creatinine amidohydrolases obtained in Example 2 and the various creatinine amidohydrolase (G179S+D175X) obtained in Comparative Example 2 were independently added to 20 mM potassium phosphate buffer solutions (pH 7.8), each of which was then divided into 2 portions. EDTA-3Na was added to one of the portions to a final concentration of 2.5 mM, while nothing was added to the other, and the 2 portions were treated at 50°C for 20 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Table 1 shows the results. As is clear from Table 2, the modified creatinine amidohydrolase of the present invention showed an improved chelating agent resistance, compared with that before modification. Further, a comparison with the wild type creatinine amidohydrolases of Comparative Example 1 reveals that the modified creatinine amidohydrolase of the present invention showed a lower Km value.

Table 2

| Amino Acid X | EDTA Resistance (%) | 11 mM/55mM ratio | Specific Activity (U/A280) |
|---|---|---|---|
| - | 8.7 | 0.62 | 144 |
| Q | 63.1 | 0.71 | 46 |
| K | 60.4 | 0.70 | 17 |
| S | 50.0 | 0.63 | 78 |
| H | 45.9 | 0.49 | 379 |
| L | 37.9 | 0.60 | 39 |
| C | 34.2 | 0.53 | 74 |
| E | 22.8 | 0.75 | 66 |
| F | 14.2 | 0.44 | 71 |
| W | 9.4 | 0.64 | 296 |

Example 5: Evaluation of modified creatinine amidohydrolase 3

The mutant creatinine amidohydrolase obtained in Example 2 and the various creatinine amidohydrolase (N43D+G179S+D175X) obtained in Comparative Example 3 were independently added to 20 mM potassium phosphate buffer solutions (pH 7.8), each of which was then divided into 2 portions. EDTA-3Na was added to one of the portions to a final concentration of 2.5 mM, while nothing was added to the other, and the 2 portions were treated at 50 °C for 20 minutes. The creatinine amidohydrolase activity was measured in accordance with the activity measurement method described above, and chelating agent resistance (%) was calculated by the formula: (activity with the addition of a chelating agent)/(activity without the addition of a chelating agent) x 100.

Table 1 shows the results. As is clear from Table 3, the modified creatinine amidohydrolase of the present invention showed an improved chelating agent resistance, compared with that before modification. Further, a comparison with the wild type creatinine amidohydrolases of Comparative Example 1 reveals that the modified creatinine amidohydrolase of the present invention showed a lower Km value. Additionally, a comparison of the specific activity values with those of Table 2 reveals that modification using N43D improves each of the specific activities.

Table 3

| Amino Acid X | EDTA Resistance (%) | 11 mM/55mM ratio | Specific Activity (U/A280) |
|---|---|---|---|
| - | 10.4 | 0.61 | 227 |
| M | 73.5 | 0.60 | 26 |
| Q | 71.2 | 0.66 | 72 |
| K | 62.5 | 0.62 | 34 |
| S | 59.6 | 0.58 | 123 |
| T | 50.2 | 0.60 | 80 |
| R | 46.6 | 0.59 | 21 |
| H | 46.3 | 0.41 | 396 |

In the Examples, each of the crude enzyme solution obtained in Example 2 was further purified in order to measure the specific activity. First, 0.5% of 5% polyethyleneimine against total volume of the crude enzyme solution was added to each of the solution, which was allowed to stand at room temperature for 30 minutes and subjected to centrifugal separation to collect the supernatant. Next, 0.65 saturated ammonium sulfate was added to each of the resulting product, which was allowed to stand at room temperature for 30 minutes and then subjected to centrifugal separation to collect the precipitate. Thereafter, the activity and absorbance at 280 nm were measured with respect to the purified enzyme sample redissolved in a 20 mM potassium phosphate buffer solution having a pH of 7.8, and the specific activity (U/A280) was calculated. Patent Document 3 discloses that the specific activity of the creatinine amidohydrolase originated from *Pseudomonas putida* (PS-7) is 1,905 U/mg. Although Comparative Example 1 produces the same product, the specific activity thereof is 350 U/A280. This is due to a difference in purity. In the Examples, a comparison is made of those that have the same purity.

Example 6: Preparation of modified creatinine amidohydrolase gene

In the same manner as in Example 1, an expression plasmid pCNH5-13 containing creatinine amidohydrolase gene was prepared.

Subsequently, mutation was performed by using a synthetic oligonucleotide (about 40 mer) containing pCNH5-13 and a triplet coding for an amino acid at the mutation introduction site in the middle of its sequence, using the QuickChange™ Site-Directed Mutagenesis Kit (product of Stratagene) in accordance with its protocol; thereby, pCNH-G179S was prepared. Using the prepared pCNH-G179S, a mutation library in which an amino acid substitution mutation was randomly introduced at the 124th position was constructed. Then, a candidate strain was obtained by screening using a change in EDTA resistance as an index, the nucleotide sequence of the candidate strain was determined, and a recombinant plasmid (pCNH-G179S+S124W) encoding a mutant creatinine amidohydrolase in which the serine at the 124th position in the amino acid sequence represented by SEQ ID NO: 2 was substituted with tryptophan was thus obtained.

Library production and screening were repeated in the same manner as above to obtain recombinant plasmids, each encoding a mutant creatinine amidohydrolase having an improved chelating agent resistance.

Example 7: Preparation of modified creatinine amidohydrolase,

Crude enzyme solution was obtained in the same manner as in Example 2. The mutant thereof was designated as G179S+S124W.

Comparative Example 4: Reparation of wild type creatinine amidohydrolase

In Comparative Example 4, Escherichia coli DH5α transformants obtained by using pCNH-G179S were subjected to the same procedure as above to obtain crude enzyme solution.

Example 8: Evaluation of modified creatinine amidohydrolase

The mutant creatinine amidohydrolase obtained in Example 7 and the various creatinine amidohydrolase obtained in Comparative Example 4 were independently added to 20 mM potassium phosphate buffer solutions (pH 7.8), each of which was then divided into 2 portions. EDTA-3Na was added to one of the portions to a final concentration of 1 mM, while nothing was added to the other, and the 2 portions were treated at 50°C for 10 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Table 4 shows the results. As is clear from Table 4, the modified creatinine amidohydrolase of the present invention showed an improved cheating agent resistance, compared with that before modification.

Table 4

| Mutation Site | Chelating Agent Resistance (%) | 11mM/55mM Ratio |
|---|---|---|
| G179S | 24.8 | 0.62 |
| G179S+S124W | 49.8 | 0.71 |

Example 9: Preparation of modified creatinine amidohydrolase gene

An expression plasmid pCNH5-13 containing creatinine amidohydrolase gene was prepared in the same manner as in Example 1.

Subsequently, mutation was performed by using a synthetic oligonucleotide (about 40 mer) containing pCNH5-13 and a triplet coding for an amino acid at the mutation introduction site in the middle of its sequence, using the QuickChange™ Site-Directed Mutagenesis Kit (product of Stratagene) in accordance with its protocol; thereby, pCNH-G179S was prepared. Using the prepared pCNH-G179S, a mutation library in which an amino acid substitution mutation was randomly introduced at the 78th position was constructed. Then, a candidate strain was obtained by screening using a change in EDTA resistance as an index, the nucleotide sequence of the candidate strain was determined, and a recombinant plasmid (pCNH-G179S+S78T) encoding a mutant creatinine amidohydrolase in which the serine at the 78th position in the amino acid sequence represented by SEQ ID NO: 2 was substituted with threonine was thus obtained.

Library production and screening were repeated in the same manner as above to obtain recombinant plasmids, each encoding a mutant creatinine amidohydrolase having an improved chelating agent resistance.

Example 10: Preparation of modified creatinine amidohydrolase

Crude enzyme solution was obtained in the same manner as in Example 2. The mutant thereof was designated as G179S+S78T.

Comparative Example 5: Preparation of wild type creatinine amidohydrolase,

In Comparative Example 5, Escherichia coli DH5$\alpha$ transformant obtained by using pCNH-G179S were subjected to the same procedure as above to obtain crude enzyme solution.

Example 11: Evaluation of modified creatinine amidohydrolase

The mutant creatinine amidohydrolase obtained in Example 10 and the various creatinine amidohydrolases obtained in Comparative Example 5 were independently added to 20 mM potassium phosphate buffer solutions (pH 7.8), each of which was then divided into 2 portions. EDTA-3Na was added to one of the portions to a final concentration of 1 mM, while nothing was added to the other, and the 2 portions were treated at 50°C for 10 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Table 5 shows the results. As is clear from Table 5, the modified creatinine amidohydrolase of the present invention showed an improved chelating agent resistance, compared with that before modification.

Table 5

| Mutation Site | Chelating Agent Resistance (%) | 11mM/55mM Ratio |
|---|---|---|
| G179S | 24.8 | 0.62 |
| G179S+S78T | 59.6 | 0.68 |
| G179S+S78R | 55.7 | 0.61 |

Example 12: Preparation of modified creatinine amidohydrolase gene

An expression plasmid pCNH5-13 containing creatinine amidohydrolase gene was prepared in the same manner as in Example 1.

Subsequently, mutation was performed by using a synthetic oligonucleotide (about 40 mer) containing pCNH5-13 and a triplet coding for an amino acid at the mutation introduction site in the middle of its sequence, using the QuickChange™ Site-Directed Mutagenesis Kit (product of Stratagene) in accordance with its protocol; thereby, pCNH-G179S was prepared. With the use of the prepared pCNH-G179S, a mutation library in which an amino acid substitution mutation was randomly introduced at the 122nd position was constructed. Then, a candidate strain was obtained by screening using a change in EDTA resistance as an index, the nucleotide sequence of the candidate strain was determined, and a recombinant plasmid (pCNH-G179S+E122S) encoding a mutant creatinine amidohydrolase in which the glutamic acid at the 122nd position in the amino acid sequence represented by SEQ ID NO: 2 was substituted with serine was thus obtained.

Library production and screening were repeated in the same manner as above to obtain recombinant plasmids, each encoding a mutant creatinine amidohydrolase having an improved chelating agent resistance.

Example 13: Preparation of modified creatinine amidohydrolase

Crude enzyme solution was obtained in the same manner as in Example 2. The mutant thereof was designated as G179S+E122S.

Comparative Example 6: Preparation of wild type creatinine amidohydrolase

In Comparative Example 6, Escherichia coli DH5α transformants obtained by using pCNH-G179S were subjected to the same procedure as above to obtain crude enzyme solution.

Example 14: Evaluation of modified creatinine amidohydrolase

The mutant creatinine amidohydrolases obtained in Example 13 and the various creatinine amidohydrolases obtained in Comparative Example 6 were independently added to 20 mM potassium phosphate buffer solutions (pH 7.8), each of which was then divided into 2 portions. EDTA-3Na was added to one of the portions to a final concentration of 1 mM, while nothing was added to the other, and the 2 portions were treated at 50°C for 10 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Table 6 shows the results. As is clear from Table 6, the modified creatinine amidohydrolase of the present invention showed an improved chelating agent resistance, compared with that before modification.

Table 6

| Mutation Site | Chelating Agent Resistance (%) | 11mM/55mM Ratio |
|---|---|---|
| G179S | 24.8 | 0.62 |
| G179S+E122s | 61.9 | 0.75 |

Example 15: Preparation of modified creatinine amidohydrolase, gene

An expression plasmid pCNH5--13 containing creatinine amidohydrolase gene was prepared in the same manner as in Example 1.

Subsequently, mutation was performed by using a synthetic oligonucleotide (about 40 mer) containing pCNH5-13 and a triplet coding for an amino acid at the mutation introduction site in the middle of its sequence, using the QuickChange™ Site-Directed Mutagenesis Kit (product of Stratagene) in accordance with its protocol; thereby, a mutation library in which an amino acid substitution mutation was randomly introduced at the 43rd position was constructed. Then, a candidate strain obtained by screening using a change in specific activity as an index, the nucleotide sequence of the candidate strain was determined, and a recombinant plasmid (pCNH-N43D) encoding a mutant creatinine amidohydrolase in which the asparagine at the 43rd position in the amino acid sequence represented by SEQ ID NO: 1 was substituted with aspartic acid was thus obtained.

Library production and screening were repeated in the same manner as above to obtain recombinant plasmids, each encoding a mutant creatinine amidohydrolase having an improved specific activity.

Example 16: Preparation of modified creatinine amidohydrolase

Crude enzyme solution was obtained in the same manner as in Example 2. The mutant thereof was designated as N43D.

Comparative Example 7: Preparation of wild type creatinine

amidohydrolase,

In Comparative Example 7, Escherichia coli DH5α transformants obtained by using pCNH5-13 were subjected to the same procedure as above to obtain crude enzyme solution before modification.

Comparative Example 8: Preparation of creatinine amidohydrolase (G179S)

In Comparative Example 8, Escherichia coli DH5α transformants obtained by using pCNH-G179S were subjected to the same procedure as above to obtain crude enzyme solution.

Comparative Example 9: Preparation of creatinine amidohydrolase (G179S+D175Q)

In Comparative Example 9, Escherichia coli DH5α transformants obtained by using pCNH-G179S+D175Q were subjected to the same procedure as above to obtain crude enzyme solution.

Comparative Example 10: Preparation of creatinine amidohydrolase (G179S+D175K)

In Comparative Example 10, Escherichia coli DH5α transformants obtained by using pCNH-G179S+D175K were subjected to the same procedure as above to obtain crude enzyme solution.

Comparative Example 11: Preparation of creatinine amidohydrolase (G179S+D175S)

In Comparative Example 11, Escherichia coli DH5α transformants obtained by using pCNH-G179S+D175S were subjected to the same procedure as above to obtain crude enzyme solution.

Comparative Example 12: Preparation of creatinine amidohydrolase (G179S+D175H)

In Comparative Example 12, Escherichia coli DH5α transformants obtained by using pCNH-G179S+D175H were subjected to the same procedure as above to obtain crude enzyme solution.

Example 17: Evaluation of modified creatinine amidonydrolase 1

The mutant creatinine amidohydrolases obtained in Example 16 and the various creatinine amidohydrolases obtained in Comparative Example 7 were independently added to 20 mM potassium phosphate buffer solutions (pH 7.8), each of which was then divided into 2 portions. EDTA-3Na was added to one of the portions to a final concentration of 5 mM, while nothing was added to the other, and the 2 portions were treated at 60°C for 20 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

The specific activity (U/OD660) was calculated by dividing the activity value (U/ml) of each of the various creatinine amidohydrolases obtained in Example 16 by the cell turbidity (OD660) before ultrasonic disruption.

Patent Document 3 discloses that the specific activity of the creatinine amidohydrolase originated from *Pseudomonas putida* (PS-7) is 1,905 U/mg. Although Comparative Example 7 produces the same product, the specific activity thereof is 53 U/0660. This is due to a difference in purity. In the Examples, a comparison is made of those that have the same purity.

Table 7 shows the results. As is clear from Table 7, the modified creatinine amidohydrolase of the present invention showed an improved specific activity, compared with that before modification. The modified creatinine amidohydrolase of the present invention also showed an improved chelating agent resistance.

Table 7

| Mutation Site | Chelating Agent Resistance (%) | 11mM/55mM Ratio | Specific Activity (U/OD660) |
|---|---|---|---|
| - | 50.1 | 0.32 | 53 |
| N43D | 74.0 | 0.30 | 78 |
| N43E | 71.7 | 0.28 | 77 |

Example 18: Evaluation of modified creatinine amidohydrolase 2

The mutant creatinine amidohydrolases obtained in Example 16 and the various creatinine amidohydrolases obtained in Comparative Example 8 were independently added to 20 mM potassium phosphate buffer solutions (pH 7.8), each of which was then divided into 2 portions. EDTA-3Na was added to one of the portions to a final concentration of 2.5 mM, while nothing was added to the other, and the 2 portions were treated at 50°C for 20 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

The specific activity (U/OD660) was calculated by dividing the activity value (U/mL) of each of the various creatinine amidohydrolases obtained in Example 16 by the cell turbidity (OD660) before ultrasonic disruption.

Table 8 shows the results. As is clear from Table 8, the modified creatinine amidohydrolase of the present invention showed an improved specific activity, compared with that before modification. Further, a comparison with the wild type creatinine amidohydrolases of Comparative Example 7 reveals that the modified creatinine amidohydrolase of the present invention showed lower Km values.

Table 8

| Mutation Site | Chelating Agent Resistance (%) | 11mM/55mM Ratio | Specific Activity (U/OD660) |
|---|---|---|---|
| G179S | 8.7 | 0.62 | 27 |
| G179S+N43D | 10.4 | 0.61 | 41 |
| G179S+N43E | - | 0.61 | 46 |

Example 19: Evaluation of modified creatinine amidohydrolase 3

The mutant creatinine amidohydrolases obtained in Example 16 and the various creatinine amidohydrolases obtained in Comparative Examples 9 to 12 were independently added to 20 mM potassium phosphate buffer solutions (pH 7.8), each of which was then divided into 2 portions. EDTA-3Na was added to one of the portions to a final concentration of 2.5 mM, while nothing was added to the other, and the 2 portions were treated at 50°C for 20 minutes. The creatinine amidohydrolase activity was measured in accordance with the activity measurement method described below, and chelating agent resistance (%) was calculated by the formula: (activity with the addition of a chelating agent)/(activity without the addition of a chelating agent) x 100.

Each of the crude enzyme solution obtained in Example 16 and Comparative Examples 9 to 12 was further purified in order to measure the specific activity. First, 0.5% of 5% polyethyleneimine against total volume of the crude enzyme solution was added to each of the solution, which was allowed to stand at room temperature for 30 minutes and then subjected to centrifugal separation to collect the supernatant. Thereafter, 0.65 saturated ammonium sulfate was added to the resulting product, which was allowed to stand at room temperature for 30 minutes and then subjected to centrifugal separation to collect the precipitate. Next, the activity and absorbance at 280 nm were measured with respect to the purified enzyme sample redissolved in a 20 mM potassium phosphate buffer solution of pH 7. 8, and the specific activity (U/A280) was calculated.

Table 9 shows the results. In the table, Nos. 1 to 4 were results of Comparative Examples 9 to 12. Comparison of the specific activity between Nos. 1 and 5, Nos. 2 and 6, Nos. 3 and 7, and Nos. 4 and 8 reveals that modification using N43D improves the specific activities as well as the chelating agent resistance.

Table 9

| No. | Mutation Site | Chelating Agent Resistance (%) | 11mM/55mM Ratio | Specific Activity (U/A280) |
|---|---|---|---|---|
| 1 | G179S+D175Q | 63.1 | 0.71 | 46 |
| 2 | G179S+D175K | 60.4 | 0.70 | 17 |
| 3 | G179S+D175S | 50.0 | 0.63 | 78 |
| 4 | G179S+D175H | 45.9 | 0.49 | 379 |
| 5 | G179S+D175Q+N43D | 71.2 | 0.66 | 72 |
| 6 | G179S+D175K+N43D | 62.5 | 0.62 | 34 |
| 7 | G179S+D175S+N43D | 59.6 | 0.58 | 123 |
| 8 | G179S+D175H+N43D | 46.3 | 0.41 | 396 |

In the Examples, the creatinine amidohydrolase activity measurement was performed as described below. One unit (U) of the enzyme activity of the present invention is defined as the amount of enzyme that produces one micromole of orange color dye per minute under the following conditions.

An enzyme product is dissolved in 50 mM of a preliminarily ice-cooled phosphate buffer having a pH of 7.5 to obtain an enzyme solution, and, immediately before analysis, the enzyme solution is diluted to 1.8 to 2.4 U/mL.

A 1.0 mL amount of a 0.1 M creatine solution (dissolved in a 50 mM phosphate buffer of pH 7.5) is introduced into a test tube and preheated at 37°C for about 5 minutes, to which 0.1 mL of the enzyme solution is added. Immediately after the reaction is properly conducted at 37°C, 0.1 mL of the reaction mixture is taken therefrom and placed into 2.0 mL of a 0.5 N NaOH solution that had been preliminarily prepared. To the resulting solution, 1.0 mL of a 1.0% picric acid solution is added, the mixture is allowed to stand at 25°C for 20 minutes, and the absorbance (ODtest) at 520 nm is measured. In a blind test, an enzyme diluting solution is added to a 0.1 M creatine solution (dissolved in a 50 mM phosphate buffer of pH 7.5) to an amount of 1.0 mL. Immediately thereafter, 0.1 mL of the reaction mixture is taken therefrom and placed into a 2.0 mL of a 0.5N NaOH-solution that had been preliminarily prepared. To the resulting solution, 1.0 mL of a 1.0% picric acid solution is added, the mixture is allowed to stand at 25°C for 20 minutes, and the absorbance (ODblank) at 520nm is measured.

$$U/mL = \Delta OD(OD\ test - OD\ blank) \times 7.33 \times dilution\ factor$$

In the Examples, as an index of the Km value, activity is measured at two levels of creatinine concentrations (final: 11 mM and 55 mM); the Km value is considered to decrease as this ratio (11 mM/55mM) increases.

R1
0.58M HEPES pH8
0.005% 4-Aminoantipyrine
0.015% Phenol
60 U/mL Creatine amidinohydrolase
12 U/mL Sarcosine oxidase
6 U/mL Peroxidase
R2 (final 55 mM)
0.25 M Creatinine
0.27 N HCl
R2 (final 11 mM)
0.05 M Creatinine
0.27 N HCl

Sixty μL of R2 and 10 μL of an enzyme liquid are added to 200 μL of R1, the mixture is allowed to react at 37°C for 10 minutes, and the absorbance variation is measured at 505 nm using a Hitachi 7060 autoanalyzer.

## Claims

1. A modified creatinine amidohydrolase having creatinine amidohydrolase activity wherein:

(1) the amino acid at position 175 in the amino acid sequence shown in SEQ ID NO: 1 of the Sequence Listing, or at a position equivalent thereto in an amino acid sequence of creatinine amidohydrolase derived from organisms other than Pseudomonas putida (PS-7), is substituted with an amino acid selected from the group consisting of threonine, glutamine, asparagine, isoleucine, serine, and leucine;
(2) the amino acid at position 175 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), is substituted with an amino acid selected from the group consisting of glutamine, lysine, serine, histidine, leucine, cysteine, glutamic acid, phenylalanine, and tryptophan;
(3) the amino acid at position 175 in the amino acid sequence shown in SEQ ID NO: 3 of the Sequence Listing, or at a position equivalent thereto in an amino acid sequence of creatinine amidohydrolase derived from organisms other than Pseudomonas putida (PS-7), is substituted with an amino acid selected from the group consisting of methionine, glutamine, lysine, serine, threonine, arginine, and histidine;
(4) an amino acid at position 124 in the amino acid sequence shown in SEQ ID NO: 2, or at a position equivalent thereto in an amino acid sequence of creatinine amidohydrolase derived from organisms other than Pseudomonas putida (PS-7), is substituted with tryptophan;
(5) an amino acid at position 78 in the amino acid sequence shown in SEQ ID NO: 2, or at a position equivalent thereto in an amino acid sequence of creatinine amidohydrolase derived from organisms other than Pseudomonas putida (PS-7), is substituted with threonine or arginine;
(6) an amino acid at position 122 in the amino acid sequence shown in SEQ ID NO: 2, or at a position equivalent thereto in an amino acid sequence of creatinine amidohydrolase derived from organisms other than Pseudomonas putida (PS-7), is substituted with serine; or
(7) an amino acid at position 43 in an amino acid sequence shown in at least one of SEQ ID NOS: 1, 2, and 7 to 10, or at a position equivalent thereto in an amino acid sequence of creatinine amidohydrolase derived from organisms other than Pseudomonas putida (PS-7), is substituted with aspartic acid or glutamic acid.

**2.** A gene encoding the modified creatinine amidohydrolase of claim 1.

**3.** A vector comprising the gene of claim 2.

**4.** A transformant transformed with the vector of claim 3.

**5.** A method for producing a modified creatinine amidohydrolase, comprising culturing the transformant of claim 4 and collecting creatinine amidohydrolase from the culture.

**6.** A reagent for measuring creatinine, comprising the modified creatinine amidohydrolase of claim 1.

**7.** A method for measuring creatinine using the modified creatinine amidohydrolase of claim 1.

**8.** A method for improving the specific activity of creatinine amidohydrolase, comprising substituting an amino acid at position 43 in an amino acid sequence shown in at least one of SEQ ID NOS: 1, 2, and 7 to 10 of the Sequence Listing, or at a position equivalent thereto in an amino acid sequence of creatinine amidohydrolase derived from organisms other than Pseudomonas putida (PS-7), with aspartic acid or glutamic acid.

**9.** A method for improving the chelating agent resistance of creatinine amidohydrolase, comprising at least one of the following steps (1) to (3):

(1) substituting an amino acid at position 124 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), with tryptophan;
(2) substituting an amino acid at position 78 in the amino acid sequence shown in SEQ ID NO: 2 of the Sequence Listing, or at a position equivalent thereto in an amino acid sequence of creatinine amidohydrolase derived from organisms other than Pseudomonas putida (PS-7), with threonine or arginine; and
(3) substituting an amino acid at position 122 in the amino acid sequence shown in SEQ ID: 2 of the Sequence Listing, or at a position equivalent thereto in an amino acid sequence of creatinine amidohydrolase derived from organisms other than Pseudomonas putida (PS-7), with serine.

**Patentansprüche**

1. Modifizierte Kreatinin-Amidohydrolase, die Kreatinin-Amidohydrolaseaktivität aufweist, wobei:

    (1) die Aminosäure an Position 175 in der in SEQ ID NO:1 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit einer Aminosäure substituiert ist, die aus der Gruppe bestehend aus Threonin, Glutamin, Asparagin, Isoleucin, Serin und Leucin ausgewählt ist;
    (2) die Aminosäure an Position 175 in der in SEQ ID NO:2 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit einer Aminosäure substituiert ist, die aus der Gruppe bestehend aus Glutamin, Lysin, Serin, Histidin, Leucin, Cystein, Glutaminsäure, Phenylalanin und Tryptophan ausgewählt ist;
    (3) die Aminosäure an Position 175 in der in SEQ ID NO:3 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit einer Aminosäure substituiert ist, die aus der Gruppe bestehend aus Methionin, Glutamin, Lysin, Serin, Threonin, Arginin und Histidin ausgewählt ist;
    (4) eine Aminosäure an Position 124 in der in SEQ ID NO:2 gezeigten Aminosäuresequenz oder an einer Position, die dieser in einer Aminosäuresequenz der Kreatinin-Amidohydrolase entspricht, die aus Organismen außer *Pseudomonas putida* (PS-7) stammt, mit Tryptophan substituiert ist;
    (5) eine Aminosäure an Position 78 in der in SEQ ID NO:2 gezeigten Aminosäuresequenz oder an einer Position, die dieser in einer Aminosäuresequenz der Kreatinin-Amidohydrolase entspricht, die aus Organismen außer *Pseudomonas putida* (PS-7) stammt, mit Threonin oder Arginin substituiert ist;
    (6) eine Aminosäure an Position 122 in der in SEQ ID NO:2 gezeigten Aminosäuresequenz oder an einer Position, die dieser in einer Aminosäuresequenz der Kreatinin-Amidohydrolase entspricht, die aus Organismen außer *Pseudomonas putida* (PS-7) stammt, mit Serin substituiert ist; oder
    (7) eine Aminosäure an Position 43 in einer in mindestens einer der in SEQ ID NOs:1, 2 und 7 bis 10 gezeigten Aminosäuresequenz oder an einer Position, die dieser in einer Aminosäuresequenz der Kreatinin-Amidohydrolase entspricht, die aus Organismen außer *Pseudomonas putida* (PS-7) stammt, mit Asparaginsäure oder Glutaminsäure substituiert ist.

2. Gen, das die modifizierte Kreatinin-Amidohydrolase nach Anspruch 1 codiert.

3. Vektor, der das Gen nach Anspruch 2 umfasst.

4. Transformante, die mit dem Vektor nach Anspruch 3 transformiert ist.

5. Verfahren zur Herstellung einer modifizierten Kreatinin-Amidohydrolase, umfassend das Züchten der Transformante nach Anspruch 4 und das Gewinnen der Kreatinin-Amidohydrolase aus der Kultur.

6. Reagens zum Messen von Kreatinin, umfassend die modifizierte Kreatinin-Amidohydrolase nach Anspruch 1.

7. Verfahren zum Messen von Kreatinin unter Verwendung der modifizierten Kreatinin-Amidohydrolase nach Anspruch 1.

8. Verfahren zur Verbesserung der spezifischen Aktivität einer Kreatinin-Amidohydrolase, umfassend das Substituieren einer Aminosäure an Position 43 in einer in mindestens einer der in SEQ ID NOs:1, 2 und 7 bis 10 gezeigten Aminosäuresequenz des Sequenzprotokolls, oder an einer Position, die dieser in einer Aminosäuresequenz der Kreatinin-Amidohydrolase entspricht, die aus Organismen außer *Pseudomonas putida* (PS-7) stammt, mit Asparaginsäure oder Glutaminsäure.

9. Verfahren zur Verbesserung der Resistenz einer Kreatinin-Amidohydrolase gegen Chelatbildner, umfassend mindestens einen der folgenden Schritte (1) bis (3):

    (1) Substituieren einer Aminosäure an Position 124 in der in SEQ ID NO:2 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit Tryptophan;
    (2) Substituieren einer Aminosäure an Position 78 in der in SEQ ID NO:2 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit Threonin oder Arginin; und
(3) Substituieren einer Aminosäure an Position 122 in der in SEQ ID NO:2 gezeigten Aminosäuresequenz des Sequenzprotokolls oder an einer Position, die dieser in einer Aminosäuresequenz der Kreatinin-Amidohydrolase entspricht, die aus Organismen außer *Pseudomonas putida* (PS-7) stammt, mit Serin.

**Revendications**

**1.** Créatinine amidohydrolase modifiée ayant une activité de créatinine amidohydrolase dans laquelle :

(1) l'acide aminé en position 175 dans la séquence d'acides aminés montrée en SEQ ID N° 1 du listage des séquences, ou en une position équivalente dans une séquence d'acides aminés de créatinine amidohydrolase dérivée d'organismes autres que *Pseudomonas putida* (PS-7), est substitué par un acide aminé sélectionné dans le groupe consistant en la thréonine, la glutamine, l'asparagine, l'isoleucine, la sérine, et la leucine ;
(2) l'acide aminé en position 175 dans la séquence d'acides aminés montrée en SEQ ID N° 2 du listage des séquences, ou en une position équivalente dans une séquence d'acides aminés de créatinine amidohydrolase dérivée d'organismes autres que *Pseudomonas putida* (PS-7), est substitué par un acide aminé sélectionné dans le groupe consistant en la glutamine, la lysine, la sérine, l'histidine, la leucine, la cystéine, l'acide glutamique, la phénylalanine, et le tryptophane ;
(3) l'acide aminé en position 175 dans la séquence d'acides aminés montrée en SEQ ID N° 3 du listage des séquences, ou en une position équivalente dans une séquence d'acides aminés de créatinine amidohydrolase dérivée d'organismes autres que *Pseudomonas putida* (PS-7), est substitué par un acide aminé sélectionné dans le groupe consistant en la méthionine, la glutamine, la lysine, la sérine, la thréonine, l'arginine, et l'histidine ;
(4) un acide aminé en position 124 dans la séquence d'acides aminés montrée en SEQ ID N° 2, ou en une position équivalente dans une séquence d'acides aminés de créatinine amidohydrolase dérivée d'organismes autres que *Pseudomonas putida* (PS-7), est substitué par le tryptophane ;
(5) un acide aminé en position 78 dans la séquence d'acides aminés montrée en SEQ ID N° 2, ou en une position équivalente dans une séquence d'acides aminés de créatinine amidohydrolase dérivée d'organismes autres que *Pseudomonas putida* (PS-7), est substitué par la thréonine ou l'arginine ;
(6) un acide aminé en position 122 dans la séquence d'acides aminés montrée en SEQ ID N° 2, ou en une position équivalente dans une séquence d'acides aminés de créatinine amidohydrolase dérivée d'organismes autres que *Pseudomonas putida* (PS-7), est substitué par la sérine ; ou
(7) un acide aminé en position 43 dans une séquence d'acides aminés montrée en au moins une de SEQ ID N° 1, 2, et 7 à 10, ou en une position équivalente dans une séquence d'acides aminés de créatinine amidohydrolase dérivée d'organismes autres que *Pseudomonas putida* (PS-7), est substitué par l'acide aspartique ou l'acide glutamique.

**2.** Gène codant pour la créatinine amidohydrolase modifiée de la revendication 1.

**3.** Vecteur comprenant le gène de la revendication 2.

**4.** Transformant transformé par le vecteur de la revendication 3.

**5.** Méthode de production d'une créatinine amidohydrolase modifiée, comprenant la mise en culture du transformant de la revendication 4 et le recueil de créatinine amidohydrolase à partir de la culture.

**6.** Réactif de mesure de créatinine, comprenant la créatinine amidohydrolase modifiée de la revendication 1.

**7.** Méthode de mesure de créatinine à l'aide de la créatinine amidohydrolase modifiée de la revendication 1.

**8.** Méthode d'amélioration de l'activité spécifique de créatinine amidohydrolase, comprenant la substitution d'un acide aminé en position 43 dans une séquence d'acides aminés montrée en au moins une de SEQ ID N° 1, 2, et 7 à 10 du listage des séquences, ou en une position équivalente dans une séquence d'acides aminés de créatinine amidohydrolase dérivée d'organismes autres que *Pseudomonas putida* (PS-7), par l'acide aspartique ou l'acide glutamique.

**9.** Méthode d'amélioration de la résistance de créatinine amidohydrolase aux agents chélatants, comprenant au moins l'une des étapes (1) à (3) suivantes :

(1) la substitution d'un acide aminé en position 124 dans la séquence d'acides aminés montrée en SEQ ID N° 2 du listage des séquences, ou en une position équivalente dans une séquence d'acides aminés de créatinine amidohydrolase dérivée d'organismes autres que *Pseudomonas putida* (PS-7), par le tryptophane ;
(2) la substitution d'un acide aminé en position 78 dans la séquence d'acides aminés montrée en SEQ ID N° 2 du listage des séquences, ou en une position équivalente dans une séquence d'acides aminés de créatinine amidohydrolase dérivée d'organismes autres que *Pseudomonas putida* (PS-7), par la thréonine ou l'arginine ; et
(3) la substitution d'un acide aminé en position 122 dans la séquence d'acides aminés montrée en SEQ ID N° 2 du listage des séquences, ou en une position équivalente dans une séquence d'acides aminés de créatinine amidohydrolase dérivée d'organismes autres que *Pseudomonas putida* (PS-7), par la sérine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0402929 A1
- JP 51115989 A
- JP 47043281 A
- JP 2527035 B
- JP 9154574 A
- JP 10215874 A
- JP 2006303451 A


### Non-patent literature cited in the description


- **YOSHIMOTO et al.** *Journal of Molecular Biology,* vol. 337 (2), 399-416
- **BEUTH et al.** *Journal of Molecular Biology,* vol. 332 (1), 287-301
- *Journal of Biochemistry,* 1979, vol. 86, 1109-1117
- *Chemical and Pharmaceutical Bulletin,* 1986, vol. 34 (1), 269-274
- *Journal of Molecular Biology,* 2004, vol. 337, 399-416
- *Journal of Molecular Biology,* 2004, vol. 332, 287-301
- *Science,* 1981, vol. 214, 1205
- *Biosci. Biotech. Biochem.,* 1995, vol. 59 (7), 1331-1332

<110> Toyoboseki Kabushiki Kaisya

<120> MODIFIED CREATININE AMIDOHYDROLASE

<130> 080036PC1

<160> 14

<170> PatentIn version 3.4

<210> 1
<211> 260
<212> PRT
<213> Pseudomonas putida

<400> 1

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly

65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Asp Ile
165 170 175

Glu His Gly Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro

210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 2
<211> 260
<212> PRT
<213> Pseudomonas putida

<400> 2

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly
65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Asp Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro
210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 3
<211> 260
<212> PRT
<213> Pseudomonas putida

<400> 3

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asp Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly
65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Asp Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro
210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 4
<211> 1474
<212> DNA
<213> Pseudomonas putida

<400> 4

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct gggacatcga gcacggcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 5
<211> 1474
<212> DNA
<213> Pseudomonas putida G179S

<400> 5

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct gggacatcga gcacagcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 6
<211> 1474
<212> DNA
<213> Pseudomonas putida G179S+N43D

<400> 6

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatggatg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct gggacatcga gcacagcggc 540

gtcttcgaga cctccctgat gctggctttg taccccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 7
<211> 260
<212> PRT
<213> Pseudomnnas putida G179S+D75Q

<400> 7

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly

65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Gln Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro

210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 8
<211> 260
<212> PRT
<213> Pseudomonas putida G179S+D175K

<400> 8

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly
65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Lys Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro
210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 9
<211> 260
<212> PRT
<213> Pseudomonas putida G179S+D175S

<400> 9

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly
65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp Ser Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro
210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 10
<211> 260
<212> PRT
<213> Pseudomonas putida G179S+D175H

<400> 10

Met Ser Lys Ser Val Phe Val Gly Glu Leu Thr Trp Lys Glu Tyr Glu
1 5 10 15

Ala Arg Val Ala Ala Gly Asp Cys Val Leu Met Leu Pro Val Gly Ala
20 25 30

Leu Glu Gln His Gly His His Met Cys Met Asn Val Asp Val Leu Leu
35 40 45

Pro Thr Ala Val Cys Lys Arg Val Ala Glu Arg Ile Gly Ala Leu Val
50 55 60

Met Pro Gly Leu Gln Tyr Gly Tyr Lys Ser Gln Gln Lys Ser Gly Gly
65 70 75 80

Gly Asn His Phe Pro Gly Thr Thr Ser Leu Asp Gly Ala Thr Leu Thr
85 90 95

Gly Thr Val Gln Asp Ile Ile Arg Glu Leu Ala Arg His Gly Ala Arg
100 105 110

Arg Leu Val Leu Met Asn Gly His Tyr Glu Asn Ser Met Phe Ile Val
115 120 125

Glu Gly Ile Asp Leu Ala Leu Arg Glu Leu Arg Tyr Ala Gly Ile Gln
130 135 140

Asp Phe Lys Val Val Val Leu Ser Tyr Trp Asp Phe Val Lys Asp Pro
145 150 155 160

Ala Val Ile Gln Gln Leu Tyr Pro Glu Gly Phe Leu Gly Trp His Ile
165 170 175

Glu His Ser Gly Val Phe Glu Thr Ser Leu Met Leu Ala Leu Tyr Pro
180 185 190

Asp Leu Val Asp Leu Asp Arg Val Val Asp His Pro Pro Ala Thr Phe
195 200 205

Pro Pro Tyr Asp Val Phe Pro Val Asp Pro Ala Arg Thr Pro Ala Pro
210 215 220

Gly Thr Leu Ser Ser Ala Lys Thr Ala Ser Arg Glu Lys Gly Glu Leu
225 230 235 240

Ile Leu Glu Val Cys Val Gln Gly Ile Ala Asp Ala Ile Arg Glu Glu
245 250 255

Phe Pro Pro Thr
260

<210> 11
<211> 1474
<212> DNA
<213> Pseudonaonas putida G179S+D175Q

<400> 11

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct ggcaaatcga gcacagcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 12
<211> 1474
<212> DNA
<213> Pseudomonas putida G179S+D175K

<400> 12

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct ggaaaatcga gcacagcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 13
<211> 1474
<212> DNA
<213> Pseudamanas putida G179S+D175S

<400> 13

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct ggagcatcga gcacagcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474

<210> 14
<211> 1474
<212> DNA
<213> Pseudomonas putida G179S+D175H

<400> 14

atgagcaaga gtgtttttgt aggtgagctg acctggaagg agtacgaggc gcgtgtcgcg 60

gcaggtgact gcgtgctcat gctgccggtc ggcgccctgg aacagcacgg ccatcacatg 120

tgcatgaacg tcgatgtgct gctgcccacg gcggtgtgca agcgggtcgc cgagcgcatt 180

ggtgcgctgg tcatgccggg gctgcagtac ggctacaagt cccagcagaa gtccggcggc 240

ggcaatcact tccccggcac caccagcctg gatggcgcca ccctgactgg cacggtgcag 300

gacatcatcc gcgagctggc gcgccatggt gcgcgtcgcc tggtactgat gaacggccac 360

tacgaaaatt ccatgttcat cgtcgaaggc atcgacctcg ccctgcgcga gctgcgctat 420

gccggcatcc aggacttcaa agtggtggtg ctctcctact gggacttcgt caaggacccg 480

gctgtgatcc agcagctcta tcccgagggc ttcctcggct ggcgcatcga gcacagcggc 540

gtcttcgaga cctccctgat gctggctttg tacccggacc tggtggacct ggaccgcgtc 600

gtcgatcacc cacctgcaac cttcccaccc tatgacgtgt ttccggtcga cccggcccgt 660

acgccggcgc cgggcactct gtcgtcggcg aatgagcaag agtgtttttg taggtgagct 720

gacctggaag gagtacgagg cgcgtgtcgc ggcaggtgac tgcgtgctca tgctgccggt 780

cggcgccctg gaacagcacg gccatcacat gtgcatgaac gtcgatgtgc tgctgcccac 840

ggcggtgtgc aagcgggtcg ccgagcgcat tggtgcgctg gtcatgccgg ggctgcagta 900

cggctacaag tcccagcaga agtccggcgg cggcaatcac ttccccggca ccaccagcct 960

ggatggcgcc accctgactg gcacggtgca ggacatcatc cgcgagctgg cgcgccatgg 1020

tgcgcgtcgc ctggtactga tgaacggcca ctacgaaaat tccatgttca tcgtcgaagg 1080

catcgacctc gccctgcgcg agctgcgcta tgccggcatc caggacttca aagtggtggt 1140

gctctcctac tgggacttcg tcaaggaccc ggctgtgatc cagcagctct atcccgaggg 1200

cttcctcggc tgggacatcg agcacggcgg cgtcttcgag acctccctga tgctggcttt 1260

gtacccggac ctggtggacc tggaccgcgt cgtcgatcac ccacctgcaa ccttcccacc 1320

ctatgacgtg tttccggtcg acccggcccg tacgccggcg ccgggcactc tgtcgtcggc 1380

gaagacggcc agccgagaga agggcgagtt gatcctggag gtctgcgtcc agggcattgc 1440

cgacgctatc cgcgaggagt tcccgcccac ctga 1474